# EUROPÄISCHE PATENTSCHRIFT

(11) **EP 2 347 755 B1**
(45) Veröffentlichungstag und Bekanntmachung des Hinweises auf die Patenterteilung: **27.06.2018**
(21) Anmeldenummer: 10156933.3
(22) Anmeldetag: 18.03.2010
(51) Int. Cl.: A61K 8/73, A61K 8/68, A61Q 19/08, A61K 8/60

(54) **Kosmetische Zusammensetzung mit Wirkstoffen zur Reduktion von Hautfalten und/oder zur Reduktion der Tiefe von Hautporen**
Cosmetic compound with active ingredients for reducing wrinkles and/or reducing the depth of pores
Préparation cosmétique dotée d'un principe actif destiné à la réduction de plis et/ou à la réduction de la profondeur des pores cutanés

(30) Priorität: 20.03.2009 DE 102009001710
(43) Veröffentlichungstag der Anmeldung: 27.07.2011
(73) Patentinhaber: Evonik Schlüchtern GmbH, 36381 Schlüchtern (DE)
(72) Erfinder: Teichmüller, Dirk, 63589, Linsengericht (DE)
(74) Vertreter: Andrae | Westendorp Patentanwälte Partnerschaft

(56) Entgegenhaltungen:
- EP-A1- 1 138 313
- WO-A1-2005/016309
- DE-A1-102006 045 389
- PILLAI R ET AL: "Beta-Glucan - A Multi-Functional Skin Care Active", COSMETIC SCIENCE TECHNOLOGY,, 1. Januar 2006 (2006-01-01), Seiten 140-145, XP009173274,

## Beschreibung

Die vorliegende Erfindung betrifft eine kosmetische Zusammensetzung mit wenigstens einem Wirkstoff zur Reduktion von Hautfalten und/oder zur Reduktion der Tiefe von Hautporen und mit einem Wirkstoffträger. Darüber hinaus betrifft die vorliegende Erfindung die Verwendung solcher Zusammensetzungen in kosmetischen Formulierungen sowie kosmetische Formulierungen, die eine solche Zusammensetzung enthalten und deren Verwendung.

Mit zunehmendem Alter verliert die menschliche Haut an Spannung und Elastizität. Gleichzeitig nimmt das Unterhautfettgewebe ab. Hierdurch können in der Haut Falten entstehen und die bereits bestehenden Falten können sich vertiefen. Die kosmetische Industrie bietet eine Vielzahl von Präparaten an, die die Hautalterung verlangsamen und der Faltenbildung entgegenwirken sollen. Viele dieser Präparate haben eine feuchtigkeitsspendende Wirkung. Diese Wirkung ist bei den verschiedenen auf dem Markt erhältlichen Präparaten oft jedoch nicht besonders zufriedenstellend und häufig auch nicht dazu geeignet, die Tiefe und Ausdehnung von bereits vorhandenen Hautfalten dauerhaft zu verringern.

Demnach besteht ein Bedarf nach einer kosmetischen Zusammensetzung bzw. nach einer eine solche Zusammensetzung enthaltenden kosmetischen Formulierung, die dazu geeignet ist, die Tiefe und Ausdehnung von Hautfalten zu reduzieren, und es war daher die Aufgabe der vorliegenden Erfindung eine entsprechend verbesserte Zusammensetzung und eine entsprechend verbesserte Formulierung bereitzustellen. Darüber hinaus sollen die erfindungsgemäße Zusammensetzung bzw. Formulierung auch in der Lage sein, die Tiefe von Hautporen zu reduzieren.

Erfindungsgemäß wird diese Aufgabe durch eine kosmetische Zusammensetzung mit wenigstens einem Wirkstoff zur Reduktion von Hautfalten und/oder zur Reduktion der Tiefe von Hautporen und mit einem Wirkstoffträger gelöst, die dadurch gekennzeichnet ist, dass die Zusammensetzung
a) als ersten Wirkstoff 0,01 bis 10 Gew.-% Hyaluronsäure,
b) als zweiten Wirkstoff 0,01 bis 10 Gew.-% β-Glucan,
c) als Wirkstoffträger 0,01 bis 10 Gew.-% Sphingolipide und/oder Galactolipide und
d) 50 bis 98 % Wasser
enthält.

Es hat sich gezeigt, dass sowohl der erste Wirkstoff, die Hyaluronsäure, als auch der zweite Wirkstoff, das β-Glucan, für sich genommen zur Reduktion von Hautfalten und/oder zur Reduktion der Tiefe von Hautporen geeignet sind. Darüber hinaus hat sich herausgestellt, dass diese beiden Wirkstoffe auch synergistisch wirken und daher zu einer besonders effektiven Reduktion von Hautfalten und/oder zur Reduktion der Tiefe von Hautporen führen, wenn diese beiden Wirkstoffe in der erfindungsgemäßen kosmetischen Zusammensetzung gemeinsam mit Sphingolipiden und/oder Galactolipiden als Wirkstoffträger eingesetzt werden.

Als Wirkstoffträger für die beiden vorgenannten Wirkstoffe enthält die Zusammensetzung entweder Sphingolipide oder Galactolipide oder Kombinationen von Sphingolipiden und Galactolipiden.

Sphingolipide sind komplexe Lipide mit Sphingosin oder einer ähnlichen Base als Grundgerüst. Sie sind wichtige Membrankomponenten pflanzlicher und tierischer Zellen und enthalten drei charakteristische Komponenten: ein Molekül Fettsäure, ein Molekül Sphingosin oder Sphingosin-Derivat und eine polare (Kopf-)Gruppe. Vorzugsweise sind die Sphingolipide, die bei der Zusammensetzung gemäß der vorliegenden Erfindung zum Einsatz kommen, unter neutralen Glycosphingolipiden, wie Cerebrosiden, Galactocerebrosiden, Glucocerebrosiden und Sulfatiden, ausgewählt.

Galactolipide sind hauptsächlich in Pflanzen vorkommende Membranlipide. Die am häufigsten vorkommenden Galactolipide in höheren Pflanzen sind Monogalactosyldiacylglycerol (MGDG) und Digalactosyldiacylglycerol (DGDG). Vorzugsweise werden in der Zusammensetzung gemäß der vorliegenden Erfindung eines oder beide der vorgenannten Galactolipide als Wirkstoffträger verwendet.

Die vorgenannten Lipide bilden ein nicht-penetrierendes Trägersystem, das bei topischer Applikation auf die Haut des Anwenders okklusiv wirkt. Bezüglich der Wirkweise der erfindungsgemäß verwendeten Lipide wird folgender Mechanismus, der jedoch keine Bindungswirkung im Sinne einer Beschränkung dieser Anmeldung haben soll, angenommen. Es wird davon ausgegangen, dass die Sphingolipide und/oder Galactolipide der erfindungsgemäßen Zusammensetzung in die oberen Hautschichten eindringen, wo sie die Wirkstoffe freigeben. Die Lipide selbst bilden in den oberen Hautschichten, insbesondere dem Stratum corneum eine okklusive Barriere, so dass die freigesetzten Wirkstoffe unterhalb dieser Barriere gehalten werden. Von hier aus können die Wirkstoffe dann in tiefere Hautschichten diffundieren, wo sie dann ihre vorteilhafte Wirkung entfalten können.

Einer der wenigstens zwei in der erfindungsgemäßen kosmetischen Zusammensetzung enthaltenen Wirkstoffe ist die Hyaluronsäure. Hyaluronsäure (auch Hyaluronan) ist ein langkettiges, lineares Glycosaminoglycan-Polysaccharid. Die makromolekulare Kette der Hyaluronsäure wird von Disaccharid-Einheiten gebildet, die wiederum aus je zwei Glucosederivaten bestehen, nämlich D-Glucuronsäure und N-Acetyl-D-glucosamin. Hierbei ist die Glucuronsäure β(1→3)-glycosidisch an das N-Acetylglucosamin geknüpft und das N-Acetylglucosamin ist auf der anderen Seite mit der nächsten Glucuronsäure in der polymeren Kette β(1→4)-glycosidisch verbunden. Eine Kette besteht typischerweise aus 2.000 bis 3.000 Disaccharideinheiten. Hyaluronsäure hat sehr gute Wasserbindungseigenschaften, was bedeutet, dass Hyaluronsäure bezogen auf ihre Masse sehr große Mengen an Wasser binden kann.

Von dem Begriff Hyaluronsäure umfaßt sind im Zusammenhang mit der vorliegenden Erfindung auch das Natrium- und das Kaliumsalz der Hyaluronsäure (Natrium- bzw. Kaliumhyaluronat), sowie weitere kosmetisch verträgliche Salze und Derivate der Hyaluronsäure, die dem Fachmann bekannt sind. Ein Stoff ist im Sinne dieser Erfindung kosmetisch verträglich, wenn er nicht toxisch ist und bei der überwiegenden Mehrzahl der potentiellen Anwender topisch anwendbar ist, ohne dass unerwünschte Nebenwirkungen, wie z. B. Hautreizungen oder Rötungen, auftreten.

Bei einer bevorzugten Ausführungsform der vorliegenden Erfindung besteht der Anteil an Hyaluronsäure wenigstens zum Teil aus hochmolekularer Hyaluronsäure mit einem Molekulargewicht in dem Bereich von 35.000 bis 2.000.000 Dalton. Diese hochmolekulare Hyaluronsäure weist besonders geeignete Wasserbindungseigenschaften auf und hat sich im Zusammenhang mit der Reduktion von Hautfalten und/oder der Reduktion der Tiefe von Hautporen als besonders vorteilhaft herausgestellt. Bei einer besonders bevorzugten Ausführungsform besteht der Anteil an Hyaluronsäure in der erfindungsgemäßen kosmetischen Zusammensetzung vollständig aus hochmolekularer Hyaluronsäure mit einem Molekulargewicht in dem Bereich von 35.000 bis 2.000.000 Dalton.

Glucane (auch Polyglucosane) sind lineare oder verzweigte Polymere der Glucose. Bei den β-Glucanen gemäß der vorliegenden Erfindung wird die Polysaccharidkette von D-Glucose-Einheiten gebildet, die β-glycosidisch verknüpft sind.

Die β-Glucane gemäß der vorliegenden Erfindung haben sehr gute Wasserbindungseigenschaften und können bezogen auf ihre Masse relativ viel Wasser aufnehmen. Als besonders vorteilhaft haben sich in diesem Zusammenhang β-Glucane, die β(1→3)- und/oder β(1→4)-glycosidisch miteinander verknüpfte Glucoseeinheiten aufweisen, und β-Glucane, die β(1→3)- und/oder β(1→6)-glycosidisch miteinander verknüpfte Glucoseeinheiten aufweisen, herausgestellt.

Vorzugsweise werden in der erfindungsgemäßen kosmetischen Zusammensetzung β-Glucane, deren Glucoseeinheiten sowohl β(1→3)- als auch β(1→4)-glycosidisch miteinander verknüpft sind (1,3/1,4-β-Glucane), oder β-Glucane, deren Glucoseeinheiten sowohl β(1→3)- als auch β(1→6)-glycosidisch miteinander verknüpft sind (1,3/1,6-β-Glucane), verwendet oder Gemische davon.

Bei den Ausführungsformen, die 1,3/1,4-β-Glucane enthalten, sind vorzugsweise die Mehrheit der Glucose-Einheiten in der Polysaccharidkette β(1→4)-glycosidisch miteinander verknüpft und nur jede dritte oder jede vierte Verknüpfung ist eine β(1→3)-glycosidische Verknüpfung. Dies führt zu einer leichten Krümmung der im übrigen im wesentlichen geraden Polysaccharidkette an der Stelle, an der die β(1→3)-glycosidische Verknüpfung vorliegt. Solche 1,3/1,4-β-Glucane finden sich beispielsweise in den Zellwänden von Getreidekörnern. Vorzugweise stammen die 1,3/1,4-β-Glucane in der erfindungsgemäßen Zusammensetzung aus Getreide bzw. Getreidekörnern. Gleichermaßen bevorzugt sind 1,3/1,4-β-Glucane, die ein Verhältnis von β(1→3)-glycosidischer Verknüpfung zu β(1→4)-glycosidischer Verknüpfung aufweisen, wie dies bei 1,3/1,4-β-Glucanen aus Getreidekörnern vorliegt.

Besonders bevorzugt sind 1,3/1,4-β-Glucane aus Körnern der Gerste (*Hordeum vulgare*), da bei diesen der Anteil der β(1→4)-glycosidischen Verknüpfung höher ist als bei 1,3/1,4-β-Glucanen aus anderen Getreidesorten, was zu einer etwas regelmäßigeren Molekülstruktur der 1,3/1,4-β-Glucane aus der Gerste führt. Gleichermaßen bevorzugt sind 1,3/1,4-β-Glucane, die ein Verhältnis von β(1→3)-glycosidischer Verknüpfung zu β(1→4)-glycosidischer Verknüpfung aufweisen, wie dies bei 1,3/1,4-β-Glucanen aus der Gerste vorliegt.

Bei den Ausführungsformen, die 1,3/1,6-β-Glucane enthalten, stammen diese vorzugsweise aus den Zellwänden von Hefen (z.B. *Saccharomyces* spec.) oder Pilzen. Gleichermaßen bevorzugt sind 1,3/1,6-β-Glucane, die ein Verhältnis von β(1→3)-glycosidischer Verknüpfung zu β(1→6)-glycosidischer Verknüpfung aufweisen, wie dies bei 1,3/1,6-β-Glucanen aus Hefen und Pilzen vorliegt. Die Gegenwart von β(1→6)-glycosidischen Verknüpfungen führt dazu, dass diese Glucane schlechter in Wasser löslich sind als die oben beschriebenen β-Glucane aus Getreide, die relativ gut in Wasser löslich sind (vorzugsweise bis zu 100% bei 80°C).

Vorzugsweise haben die in der erfindungsgemäßen Zusammensetzung verwendeten β-Glucane ein Molekulargewicht in dem Bereich von 10.000 bis 500.000 Da. Besonders bevorzugt haben die in der erfindungsgemäßen Zusammensetzung verwendeten β-Glucane ein Molekulargewicht in dem Bereich von 20.000 bis 200.000 Da.

Bei einer besonders bevorzugten Ausführungsform der erfindungsgemäßen Zusammensetzung enthält diese:
a) 0,01 bis 10 Gew.-% Hyaluronsäure,
b) 0,01 bis 10 Gew.-% β-Glucan,
c) 0,01 bis 10 Gew.-% Sphingolipide und/oder Galactolipide und
d) 50 bis 98 Gew.-% Wasser.

Wahlweise enthält die erfindungsgemäße Zusammensetzung neben den Bestandteilen a) bis d) auch einen Anteil von 0,5 bis 30 Gew.-% Ethanol oder eines anderen Konservierungsmittels.

Bei einer bevorzugten Ausführungsform der erfindungsgemäßen Zusammensetzung liegt der Wirkstoffträger in Form von Vesikeln vor, die von den Sphingolipiden und/oder Galactolipiden gebildet werden. Die Vesikel können zusätzlich weitere Bestandteile enthalten, die die Vesikel stabilisieren oder deren physiko-chemischen Eigenschaften in anderer Weise positiv beeinflussen. Hierfür kommen beispielsweise Fettsäuren oder andere Lipide in Betracht, die sich in die Substanz aus Sphingolipiden und/oder Galactolipiden eingliedern. Vorzugsweise übersteigt der Anteil dieser zusätzlichen Stoffe in dem Wirkstoffträger aus Sphingolipiden und/oder Galactolipiden den Anteil von höchstens 10 Gew.-% nicht. Demnach besteht der Wirkstoffträger vorzugsweise zu wenigstens 90 Gew.-% aus Sphingolipiden und/oder Galactolipiden. Noch bevorzugter besteht der Wirkstoffträger zu wenigstens 95 Gew.-% aus Sphingolipiden und/oder Galactolipiden.

Bei den Ausführungsformen der vorliegenden Erfindung, bei denen der Wirkstoffträger in Form von Vesikeln vorliegt, können diese Vesikel eine einfache Hülle aus einer Lipideinzelschicht-Membran aufweisen. Vorzugsweise weisen die Wirkstoffträgervesikel in der erfindungsgemäßen Zusammensetzung eine Lipiddoppelschicht-Membran auf. Unabhängig von der Anzahl ihrer Membranschichten weisen die Vesikel eine mittlere Teilchengröße in dem Bereich von 50 bis 1.000 nm auf.

Vorzugsweise sind sowohl die Hyaluronsäure als auch das β-Glucan in die Substanz des Wirkstoffträgers aus Sphingolipiden und/oder Galactolipiden eingebettet, oder zumindest mit dieser assoziiert. Bei den Ausführungsformen mit vesikelförmigen Wirkstoffträgern sind vorzugsweise sowohl die Hyaluronsäure als auch das β-Glucan in den Vesikeln enthalten. Bei alternativen Ausführungsformen ist nur einer der beiden Wirkstoffe in die Wirkstoffträgersubstanz eingebettet, mit dieser assoziiert oder darin enthalten, während der andere Wirkstoff und ggf. weitere Wirkstoffe in der Zusammensetzung frei oder mit einer anderen Wirkstoffträgersubstanz assoziiert ist/sind bzw. an dieser gebunden oder darin enthalten vorliegt/en.

Neben den beiden zwingend enthaltenen Wirkstoffen für Hyaluronsäure und β-Glucan können auch weitere Wirkstoffe in der erfindungsgemäßen Zusammensetzung enthalten sein, die entweder ebenfalls der Reduktion von Hautfalten und/oder zur Reduktion der Tiefe von Hautporen dienen oder die eine zweite hiervon unabhängige oder ergänzende Wirkung aufweisen.

Der pH-Wert der erfindungsgemäßen kosmetischen Zusammensetzung liegt vorzugsweise in dem Bereich von 5 bis 8.

Der beanspruchte Gegenstand der vorliegenden Erfindung umfaßt auch die Verwendung der oben beschriebenen erfindungsgemäßen Zusammensetzung zur Herstellung einer kosmetischen Formulierung sowie eine kosmetische Formulierung, die die oben beschriebene erfindungsgemäße Zusammensetzung enthält. Vorzugsweise ist diese Formulierung für die topische Applikation. Unter topischer Applikation wird im Zusammenhang mit der vorliegenden Erfindung eine örtliche Verabreichung von Wirkstoffen durch Aufbringen der Wirkstoffe auf die Haut verstanden.

Erforderlichenfalls enthält die Formulierung neben den für die erfindungsgemäße Zusammensetzung beschriebenen Bestandteilen Hilfs- und Zusatzstoffe, wie sie üblicherweise bei kosmetischen Präparaten Anwendung finden. Hierunter sind im Zusammenhang mit der vorliegenden Erfindung solche Stoffe zu verstehen, die auf die physikalischen Eigenschaften der Zusammensetzung oder der Formulierung und deren Stabilität einwirken und/oder der Konservierung der Zusammensetzung bzw. der Formulierung dienen, wie z. B. Öle, Alkohole, Gelbildner, Puffer, Konservierungsmittel, Emulgiermittel, Lösungsvermittler, Stabilisatoren, Verdicker oder Komplexbildner.

Die erfindungsgemäße Zusammensetzung kann in allen für die topische Applikation geeigneten Formulierungen vorliegen, beispielsweise in Form eines Gels, einer Creme, einer Salbe, eines Sprays oder einer Lotion. Dazu kann die erfindungsgemäße Zusammensetzung in eine Trägermatrix eingearbeitet werden. Bei der Trägermatrix kann es sich um Gelformulierungen, Cremeformulierungen, Lotionen, Maskenanwendungen etc. handeln.

Für Zwecke der ursprünglichen Offenbarung wird darauf hingewiesen, dass sämtliche Merkmale, wie sie sich aus der vorliegenden Beschreibung, den Ansprüchen und den Figuren für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder chemische, physikalisch-chemische, kosmetische oder dermatologische Gegebenheiten derartige Kombinationen unmöglich oder sinnlos machen. Auf die umfassende, explizite Darstellung sämtlicher denkbaren Merkmalskombinationen wird hier nur der Kürze und der Lesbarkeit der Beschreibung wegen verzichtet.

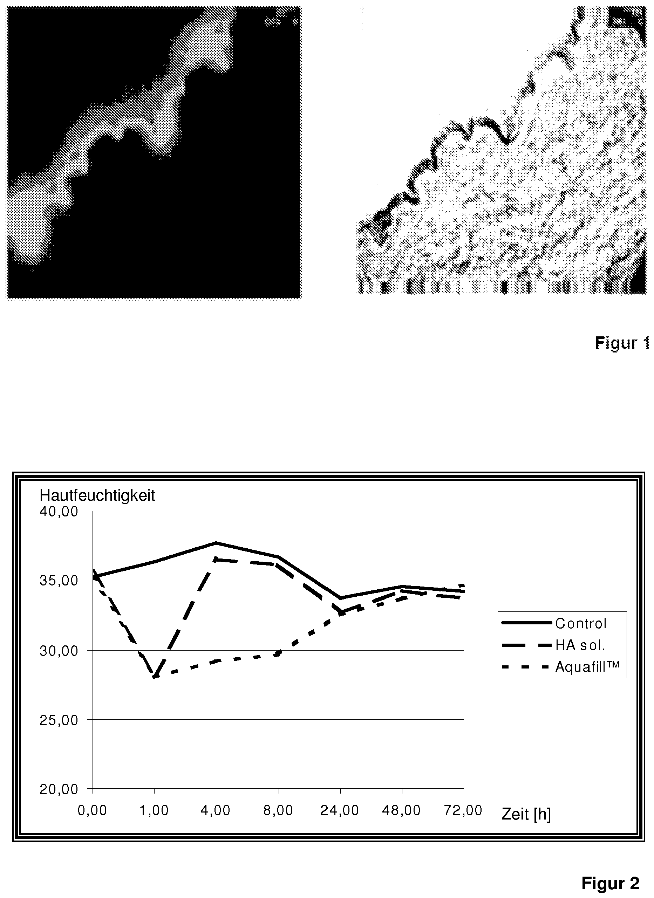

Ein Beispiel für die mögliche Kombination von Merkmalen im Sinne des vorangehenden Absatzes stellt die unten beschriebene Beispielsformulierung dar, deren Wirksamkeit bei der Reduktion von Hautfalten und/oder der Reduktion der Tiefe von Hautporen anhand der Figuren 1 bis 4 veranschaulicht wird. Hierbei zeigen:
- Figur 1:: mikroskopische Querschnittsansichten von Hautbiopsien, die mit einer erfindungsgemäßen kosmetischen Formulierung behandelt wurden,
- Figur 2:: eine graphische Darstellung von Feuchtigkeitsmessungen zu verschiedenen Meßzeitpunkten bei einem *in vivo*-Test einer erfindungsgemäßen Formulierung, einer Hyaluronsäurelösung und einer Kontrolle,
- Figur 3:: eine graphische Darstellung des Ergebnisses einer *in vivo*-Untersuchung der Haut von Probanden im Hinblick auf das Volumen und die Tiefe der Hautporen und
- Figur 4:: eine graphische Darstellung der faltenverringernden Wirkung einer erfindungsgemäßen kosmetischen Formulierung in einer *in vivo*-Studie.

### Formulierungsbeispiel

In den nachfolgend vorgestellten und diskutierten Versuchen wurde eine Beispielformulierung verwendet, die eine erfindungsgemäße kosmetische Zusammensetzung enthält. Diese Beispielformulierung enthält im einzelnen:

| | |
|---|---|
| ad 100 Gew.-% | Wasser dest. |
| 1,17 Gew.-% | Acritamer 940 |
| 4,14 Gew.-% | Natriumhydroxid (10 %) |
| 4,50 Gew.-% | Atlas 2330 |
| 0,45 Gew.-% | Euxyl K702 |
| 0,025 Gew.-% | Natrium-Hyaluronat (1.000.000 bis 1.500.000 Da) |
| 0,125 Gew.-% | 1,3/1,4-β-Glucan (aus der Gerste; 20.000 bis 200.000 Da) |
| 0,15 Gew.-% | Glycosphingolipide |
| 0,825 Gew.-% | Ethanol |

Die oben beschriebene Beispielformulierung wurde in den folgenden vier Studien untersucht.

### 1. Ex vivo-Fülleffekt:

Die erfindungsgemäße kosmetische Formulierung wurde mit dem hydrophilen Fluoreszenzmarker Carboxyfluorescein beladen und auf Hautbiopsien aufgetragen. Nach einer Einwirkzeit von drei Stunden wurden die Biopsien gereinigt, und mittels konfokaler Laserscan-Mikroskopie wurde der Faltenfülleffekt der Beispielformulierung bestimmt.

Aus den in Figur 1 dargestellten mikroskopischen Aufnahmen ist zu erkennen, dass die ursprünglich vorhandenen Hautfalten durch die fluoreszierende kosmetische Formulierung gemäß der vorliegenden Erfindung reduziert wurden.

### 2. Untersuchung der Feuchtigkeitskonservierung

Im Rahmen einer *in vivo*-Studie wurde die erfindungsgemäße kosmetische Formulierung auf die Haut von Probanden aufgetragen. Anschließend wurde über einen Zeitraum von insgesamt 72 Stunden die Hautfeuchtigkeit an der Hautoberfläche mit einem Corneometer (Courage & Khazaka) bestimmt. Je effektiver die Filmbildung auf der Haut, desto länger wurde die Feuchtigkeit in der Haut konserviert und war somit an der Oberfläche nicht mehr meßbar.

Verglichen wurden die Studienergebnisse mit einer kosmetischen Formulierung, die als Wirkstoff nur hochmolekulare Hyaluronsäure enthielt (HA sol). Die Kontrolle (Control) repräsentiert die Probanden, die unbehandelt blieben. Das Ergebnis der Behandlung mit der erfindungsgemäßen kosmetischen Formulierung ist mit der Bezeichnung "Aquafill" bezeichnet.

Die Versuchsergebnisse zeigen deutlich, dass der Hautfeuchtigkeitszustand der unbehandelten Haut um den Wert von 35 schwankt. Die Behandlung mit der kosmetischen Formulierung, die nur Hyaluronsäure enthielt, führte dazu, dass die Haut über einen Zeitraum von etwa 2 Stunden signifikant weniger Feuchtigkeit verlor als die Haut der Probanden in der Kontrollgruppe. Danach glich sich der Hautfeuchtigkeitsverlust bei den mit dieser Formulierung behandelten Probanden den entsprechenden Werten zu den folgenden Zeitpunkten in der Kontrollgruppe an.

Auch die Behandlung mit der erfindungsgemäßen Formulierung führte zu einer signifikanten feuchtigkeitskonservierenden Wirkung. Im Gegensatz zu der Formulierung, die nur Hyaluronsäure enthielt, hielt diese Wirkung jedoch auch noch nach zwei Stunden und sogar noch nach etwa zwölf Stunden an. Erst nach etwa 24 Stunden war der Hautfeuchtigkeitszustand der mit der erfindungsgemäßen Formulierung behandelten Gruppe in dem Bereich der Kontrollgruppe angelangt.

Dies zeigt, dass die erfindungsgemäße Formulierung nicht nur gegenüber der Kontrollgruppe zu einer Verbesserung führt, sondern dass sie auch im Vergleich mit der Formulierung, die nur Hyaluronsäure enthält, eine deutlich bessere feuchtigkeitskonservierende Wirkung aufweist, da diese Wirkung signifikant länger anhält.

### 3. Verkleinerung von Hautporen

Im Rahmen einer *in vivo*-Studie wurde die erfindungsgemäße kosmetische Formulierung auf die Haut von Probanden aufgetragen. Nach der Applikation wurden das Volumen und die Tiefe der Hautporen gemessen und hierbei auch die tiefsten Poren bestimmt (SELS - Surface Evaluation of Living Skin).

In Figur 1 ist zu erkennen, dass die Behandlung mit der erfindungsgemäßen kosmetischen Formulierung zu einer deutlichen Glättung der Haut führt (Figur 1a) = vorher = unbehandelt; Figur 1b) = nachher = behandelt). In Zahlen ausgedrückt sieht das Ergebnis dieser Untersuchung wie folgt aus:

| | vorher | nachher |
|---|---|---|
| Gesamtvolumen der Poren [mm²]* | 1,37 | 1,01 |
| mittlere Tiefe der Poren [µm] | 51,1 | 41,8 |
| tiefste Stelle [µm] | 77,0 | 59,0 |

| | | |
|---|---|---|
| * Die Einheit des Porenvolumens ist mm², da bei der Auswertung eine 2D-Fläche mit Graustufen aus einer Bildverarbeitungssoftware multipliziert wird, wobei die die jeweiligen topographischen Höhen repräsentierenden Graustufen einheitslos sind. | | |

### 4. Faltenreduktion

Im Rahmen einer *in vivo*-Studie an 20 Probanden wurde die erfindungsgemäße kosmetische Formulierung auf die Haut der Probanden appliziert. Die Faltenreduktion wurde drei und 24 Stunden nach einmaliger Applikation bestimmt (PRIMOS). Darüber hinaus wurde die Formulierung auch in einer Langzeitstudie über 28 Tage eingesetzt und hierbei einmal täglich aufgetragen. Die Faltenreduktion wurde hier jeweils nach 14 und 28 Tagen bestimmt.

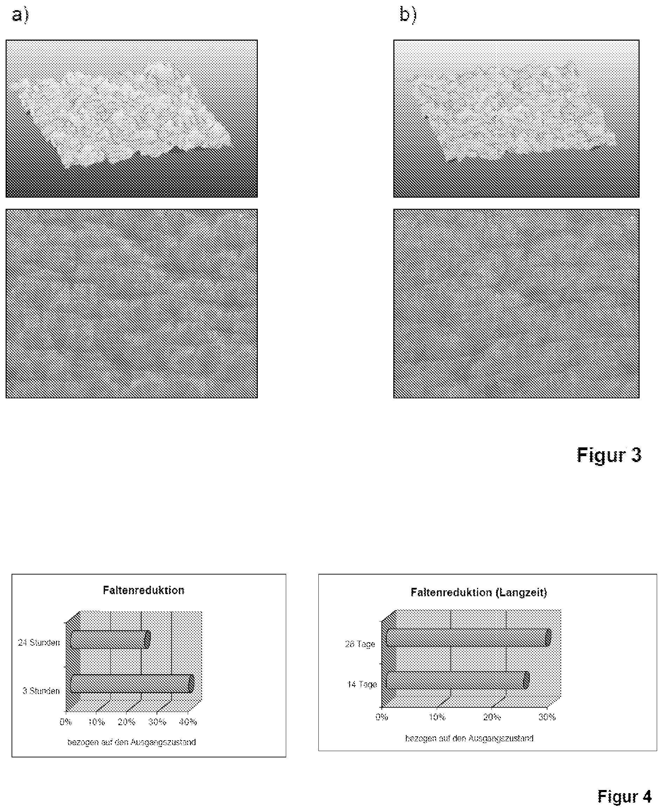

Aus den graphischen Darstellungen in Figur 4 ist zu erkennen, dass bei einmaligem Auftragen (Abbildung a) nach drei Stunden eine Verringerung der Faltentiefe um 40 % erfolgte. Nach 24 Stunden betrug diese Verringerung immer noch über 20 %. Bei einer täglichen Verabreichung über mehrere Tage (Abbildung b) wurde nach 14 Tagen eine Verringerung der Faltentiefe um über 20 % und nach 28 Tagen um etwa 30 % erreicht.

## Patentansprüche

1. Kosmetische Zusammensetzung mit wenigstens einem Wirkstoff zur Reduktion von Hautfalten und/oder zur Reduktion der Tiefe von Hautporen und mit einem Wirkstoffträger, **dadurch gekennzeichnet, dass** die Zusammensetzung
a) als ersten Wirkstoff 0,01 bis 10 Gew.-% Hyaluronsäure,
b) als zweiten Wirkstoff 0,01 bis 10 Gew.-% β-Glucan,
c) als Wirkstoffträger 0,01 bis 10 Gew.-% Sphingolipide und/oder Galactolipide und
d) 50 bis 98 Gew.-% Wasser
enthält.

2. Zusammensetzung nach Anspruch 1, **dadurch gekennzeichnet, dass** die Hyaluronsäure ein Molekulargewicht von 35.000 bis 2.000.000 Dalton aufweist.

3. Zusammensetzung nach Anspruch 2, **dadurch gekennzeichnet, dass** das β-Glucan ausgewählt ist unter 1,3/1,4-β-Glucanen und 1,3/1,6-β-Glucanen oder Gemischen davon.

4. Zusammensetzung nach einem der Ansprüche 1 bis 3, **dadurch gekennzeichnet, dass** die Sphingolipide unter neutralen Glycosphingolipiden, wie Cerebrosiden, Galactocerebrosiden, Glucocerebrosiden und Sulfatiden, ausgewählt sind.

5. Zusammensetzung nach einem der Ansprüche 1 bis 4, **dadurch gekennzeichnet, dass** die Galactolipide unter Monogalactosyldiacylglycerol und Digalactosyldiacylglycerol ausgewählt sind.

6. Zusammensetzung nach einem der Ansprüche 1 bis 5, **dadurch gekennzeichnet, dass** sie einen Anteil von 0,5 bis 30 Gew.-% Ethanol oder eines anderen Konservierungsmittels enthält.

7. Zusammensetzung nach einem der Ansprüche 1 bis 6, **dadurch gekennzeichnet, dass** der Wirkstoffträger in einem wasserhaltigen Medium in Form von Vesikeln, die von Sphingolipiden und/oder Galactolipiden gebildet werden, vorliegt.

8. Zusammensetzung nach Anspruch 7, **dadurch gekennzeichnet, dass** die Vesikel eine Lipiddoppelschicht-Membran aufweisen.

9. Zusammensetzung nach einem der Ansprüche 7 oder 8, **dadurch gekennzeichnet, dass** die Vesikel eine mittlere Teilchengröße von 50 bis 1000 nm aufweisen.

10. Zusammensetzung nach einem der Ansprüche 7 bis 9, **dadurch gekennzeichnet, dass** die Hyaluronsäure und das β-Glucan in den Vesikeln enthalten sind.

11. Verwendung einer Zusammensetzung nach einem der Ansprüche 1 bis 10 zur Herstellung einer kosmetischen Formulierung zur Reduktion von Hautfalten und/oder zur Reduktion der Tiefe von Hautporen.

12. Kosmetische Formulierung zur Reduktion von Hautfalten und/oder zur Reduktion der Tiefe von Hautporen, wobei die Formulierung eine Zusammensetzung nach einem der Ansprüche 1 bis 10 enthält.

13. Verwendung nach Anspruch 11 oder Formulierung nach Anspruch 12, **dadurch gekennzeichnet, dass** die Formulierung für die topische Applikation ist.

14. Zusammensetzung nach einem der Ansprüche 1 bis 10, Verwendung nach einem der Ansprüche 11 oder 13 oder Formulierung nach Anspruch 12, **dadurch gekennzeichnet, dass** die Zusammensetzung und/oder die Formulierung neben Wirkstoffen und Wirkstoffträger auch Hilfs- und Zusatzstoffe enthält.

## Claims

1. Cosmetic composition comprising at least one active ingredient for the reduction of skin wrinkles and/or for the reduction of the depth of skin pores and comprising an active ingredient carrier, **characterized in that** the composition contains:
a) 0.01 to 10 wt% of hyaluronic acid as first active ingredient,
b) 0.01 to 10 wt% of β-glucan as second active ingredient,
c) 0.01 to 10 wt% of sphingolipids and/or galactolipids as active ingredient carrier, and
d) 50 to 98 wt% of water.

2. Composition according to Claim 1, **characterized in that** the hyaluronic acid has a molecular weight of 35 000 to 2 000 000 daltons.

3. Composition according to Claim 2, **characterized in that** the β-glucan is selected from 1,3-/1,4-β-glucans and 1,3-/1,6-β-glucans or mixtures thereof.

4. Composition according to one of Claims 1 to 3, **characterized in that** the sphingolipids are selected from neutral glycosphingolipids, such as cerebrosides, galactocerebrosides, glucocerebrosides and sulfatides.

5. Composition according to one of Claims 1 to 4, **characterized in that** the galactolipids are selected from monogalactosyldiacylglycerol and digalactosyldiacylglycerol.

6. Composition according to one of Claims 1 to 5, **characterized in that** it contains a proportion of 0.5 to 30 wt% of ethanol or of another preservative.

7. Composition according to one of Claims 1 to 6, **characterized in that** the active ingredient carrier is present in an aqueous medium in the form of vesicles that are formed of sphingolipids and/or galactolipids.

8. Composition according to Claim 7, **characterized in that** the vesicles have a lipid bilayer membrane.

9. Composition according to either of Claims 7 and 8, **characterized in that** the vesicles have a mean particle size of 50 to 1000 nm.

10. Composition according to one of Claims 7 to 9, **characterized in that** the hyaluronic acid and the β-glucan are contained in the vesicles.

11. Use of a composition according to one of Claims 1 to 10 for the preparation of a cosmetic formulation for the reduction of skin wrinkles and/or for the reduction of the depth of skin pores.

12. Cosmetic formulation for the reduction of skin wrinkles and/or for the reduction of the depth of skin pores, wherein the formulation contains a composition according to one of Claims 1 to 10.

13. Use according to Claim 11 or formulation according to Claim 12, **characterized in that** the formulation is for topical application.

14. Composition according to one of Claims 1 to 10, use according to either of Claims 11 and 13, or formulation according to Claim 12, **characterized in that** the composition and/or the formulation also contains excipients and additives alongside active ingredients and active ingredient carrier.

## Revendications

1. Composition cosmétique contenant au moins un agent actif pour la réduction des rides de la peau et/ou pour la réduction de la profondeur des pores de la peau, et contenant un support d'agent actif, **caractérisée en ce que** la composition contient :
a) en tant que premier agent actif, 0,01 à 10 % en poids d'acide hyaluronique,
b) en tant que deuxième agent actif, 0,01 à 10 % en poids de β-glucane,
c) en tant que support d'agent actif, 0,01 à 10 % en poids de sphingolipides et/ou de galactolipides, et
d) 50 à 98 % en poids d'eau.

2. Composition selon la revendication 1, **caractérisée en ce que** l'acide hyaluronique présente un poids moléculaire de 35 000 à 2 000 000 Dalton.

3. Composition selon la revendication 2, **caractérisée en ce que** le β-glucane est choisi parmi les 1,3/1,4-β-glucanes et les 1,3/1,6-β-glucanes ou leurs mélanges.

4. Composition selon l'une quelconque des revendications 1 à 3, **caractérisée en ce que** les sphingolipides sont choisis parmi les glycosphingolipides neutres, tels que les cérébrosides, les galactocérébrosides, les glucocérébrosides et les sulfatides.

5. Composition selon l'une quelconque des revendications 1 à 4, **caractérisée en ce que** les galactolipides sont choisis parmi le monogalactosyldiacylglycérol et le digalactosyldiacylglycérol.

6. Composition selon l'une quelconque des revendications 1 à 5, **caractérisée en ce qu'**elle contient une proportion de 0,5 à 30 % en poids d'éthanol ou d'un autre conservateur.

7. Composition selon l'une quelconque des revendications 1 à 6, **caractérisée en ce que** le support d'agent actif se présente dans un milieu contenant de l'eau sous la forme de vésicules, qui sont formées par des sphingolipides et/ou des galactolipides.

8. Composition selon la revendication 7, **caractérisée en ce que** les vésicules présentent une membrane lipidique à double couche.

9. Composition selon l'une quelconque des revendications 7 ou 8, **caractérisée en ce que** les vésicules présentent une taille de particule moyenne de 50 à 1 000 nm.

10. Composition selon l'une quelconque des revendications 7 à 9, **caractérisée en ce que** l'acide hyaluronique et le β-glucane sont contenus dans les vésicules.

11. Utilisation d'une composition selon l'une quelconque des revendications 1 à 10 pour la fabrication d'une formulation cosmétique pour la réduction des rides de la peau et/ou pour la réduction de la profondeur des pores de la peau.

12. Formulation cosmétique pour la réduction des rides de la peau et/ou pour la réduction de la profondeur des pores de la peau, dans laquelle la formulation contient une composition selon l'une quelconque des revendications 1 à 10.

13. Utilisation selon la revendication 11 ou formulation selon la revendication 12, **caractérisée en ce que** la formulation est pour l'application topique.

14. Composition selon l'une quelconque des revendications 1 à 10, utilisation selon l'une quelconque des revendications 11 ou 13 ou formulation selon la revendication 12, **caractérisée en ce que** la composition et/ou la formulation contiennent également des adjuvants et des additifs en plus des agents actifs et des supports d'agents actifs.
